# EUROPEAN PATENT APPLICATION

(11) **EP 4 708 453 A2**
(43) Date of publication of application: **11.03.2026**
(21) Application number: 25187700.7
(22) Date of filing: 07.07.2025
(51) Int. Cl.: H01M 10/54

(54) **BATTERY RECYCLING SYSTEM, CONTROL METHOD, AND CONTROL PROGRAM**

(30) Priority: 08.08.2024 JP 2024131782
(71) Applicant: TOYOTA JIDOSHA KABUSHIKI KAISHA, Toyota-shi, Aichi-ken 471-8571 (JP)
(72) Inventor: KATO, Yasushi, Toyota-shi, Aichi-ken, (JP); MURAMATSU, Kenichiro, Toyota-shi, Aichi-ken, (JP); NORITAKE, Kazuki, Nisshin-shi, Aichi-ken, (JP); ISHIMOTO, Junichi, Okazaki-shi, Aichi-ken, (JP)
(74) Representative: Winter, Brandl - Partnerschaft mbB

(57) **Abstract**

A battery recycling system (1) includes: a discharging device (11) configured to discharge a secondary battery (TG) to be recycled; a primary crushing device (12) configured to crush a case of the secondary battery that has been discharged to cause an electrode material of the secondary battery to be exposed; an electrolytic solution recovery device (13) configured to heat the secondary battery under a reduced-pressure environment to recover an electrolytic solution contained in the electrode material; a secondary crushing device (14) configured to further crush the secondary battery whose electrolytic solution has been recovered; a sorting device (15) configured to sort crushed objects of the secondary battery crushed by the secondary crushing device (14); and an adjustment device (16) configured to adjust an atmosphere in each of the primary crushing device, the electrolytic solution recovery device, the secondary crushing device, and the sorting device to an N2 atmosphere.

## Description

### BACKGROUND

The present disclosure relates to a battery recycling system, a control method, and a control program.

When a secondary battery such as a nickel metal hydride battery or a lithium-ion battery is recycled, it is required to accurately sort crushed objects generated by crushing the secondary battery while reducing emission of CO2 (carbon dioxide). For example, Patent Literature 1 discloses a crushing and classifying device for improving a collection ability of an active material included in an electrode material of a secondary battery.

[Patent Literature 1] Japanese Unexamined Patent Application Publication No. 2024-73986

### SUMMARY

There is a problem in Patent Literature 1 that, although the secondary battery after being discharged is crushed, due to the presence of the electrolytic solution remaining in the secondary battery, a separator in the secondary battery is melted due to a short circuit of the crushed secondary battery and the melted separator sticks to the electrode material, which prevents the crushed objects of the secondary battery from being accurately sorted.

The present disclosure has been made in view of the aforementioned background, and an object of the present disclosure is to provide a battery recycling system, a control method, and a control program capable of accurately sorting crushed objects of a secondary battery and efficiently recycling them while reducing emission of CO2 by non-torrefaction.

A battery recycling system according to the present disclosure further includes: a discharging device configured to discharge a secondary battery to be recycled; a primary crushing device configured to crush a case of the secondary battery that has been discharged to cause an electrode material of the secondary battery to be exposed; an electrolytic solution recovery device configured to heat the secondary battery whose electrode material has been exposed under a reduced-pressure environment to recover an electrolytic solution contained in the electrode material; a secondary crushing device configured to further crush the secondary battery whose electrolytic solution has been recovered; a sorting device configured to sort crushed objects of the secondary battery crushed by the secondary crushing device; and an adjustment device configured to adjust an atmosphere in each of the primary crushing device, the electrolytic solution recovery device, the secondary crushing device, and the sorting device to an N2 atmosphere. The battery recycling system according to the present disclosure adjusts the atmosphere in each of the primary crushing device, the electrolytic solution recovery device, the secondary crushing device, and the sorting device to the N2 atmosphere and reduces the oxygen concentration, thereby preventing heat generation due to a short circuit of the crushed secondary battery more reliably. Accordingly, the battery recycling system according to the present disclosure is able to prevent the separator in the crushed secondary battery from melting and prevent the melted separator from sticking to the electrode material, and further prevent oxidation of the powdery crushed objects of the secondary battery, whereby it is possible to accurately sort the crushed objects of the secondary battery. That is, the battery recycling system according to the present disclosure is able to accurately sort the crushed objects of the secondary battery and efficiently recycle the crushed objects while reducing emission of CO2 by non-torrefaction.

The adjustment device may adjust an amount of N2 supplied in such a way that the oxygen concentration in each of the primary crushing device, the electrolytic solution recovery device, the secondary crushing device, and the sorting device becomes equal to or smaller than 2%.

The adjustment device may adjust an amount of N2 supplied in such a way that the oxygen concentration in each of the primary crushing device and the electrolytic solution recovery device becomes equal to or small than 2% and the oxygen concentration in each of the secondary crushing device and the sorting device becomes equal to or smaller than 6%.

Each of the primary crushing device, the electrolytic solution recovery device, the secondary crushing device, and the sorting device may start an operation when the oxygen concentration therein has been adjusted to be the one required by the adjustment device.

The secondary battery is a lithium-ion battery or a nickel metal hydrate battery.

In a control method of a battery recycling system according to the present disclosure, the battery recycling system: discharges, using a discharging device, a secondary battery to be recycled; crushes, using a primary crushing device, a case of the secondary battery that has been discharged to cause an electrode material of the secondary battery to be exposed; heats, using an electrolytic solution recovery device, the secondary battery whose electrode material has been exposed under a reduced-pressure environment to recover an electrolytic solution contained in the electrode material; further crushes, using a secondary crushing device, the secondary battery whose electrolytic solution has been recovered; and sorts, using a sorting device, crushed objects of the secondary battery crushed by the secondary crushing device, and an atmosphere in each of the primary crushing device, the electrolytic solution recovery device, the secondary crushing device, and the sorting device is adjusted to an N2 atmosphere. In the control method of the battery recycling system according to the present disclosure, the atmosphere in each of the primary crushing device, the electrolytic solution recovery device, the secondary crushing device, and the sorting device is adjusted to the N2 atmosphere and the oxygen concentration is reduced, thereby preventing heat generation due to a short circuit of the crushed secondary battery more reliably. Accordingly, with the control method of the battery recycling system according to the present disclosure, it is possible to prevent the separator in the crushed secondary battery from melting and prevent the melted separator from sticking to the electrode material, and further prevent oxidation of the powdery crushed objects of the secondary battery, whereby it is possible to accurately sort the crushed objects of the secondary battery. That is, with the control method of the battery recycling system according to the present disclosure, it is possible to accurately sort the crushed objects of the secondary battery and efficiently recycle the crushed objects while reducing emission of CO2 by non-torrefaction.

A control program according to the present disclosure is a control program for causing a computer to execute: processing for discharging, using a discharging device, a secondary battery to be recycled; processing for crushing, using a primary crushing device, a case of the secondary battery that has been discharged to cause an electrode material of the secondary battery to be exposed; processing for heating, using an electrolytic solution recovery device, the secondary battery whose electrode material has been exposed under a reduced-pressure environment to recover an electrolytic solution contained in the electrode material; processing for further crushing, using a secondary crushing device, the secondary battery whose electrolytic solution has been recovered; and processing for sorting, using a sorting device, crushed objects of the secondary battery crushed by the secondary crushing device, and the computer is caused to further execute processing for adjusting an atmosphere in each of the primary crushing device, the electrolytic solution recovery device, the secondary crushing device, and the sorting device to an N2 atmosphere. With the control program according to the present disclosure, the atmosphere in each of the primary crushing device, the electrolytic solution recovery device, the secondary crushing device, and the sorting device is adjusted to the N2 atmosphere and the oxygen concentration is reduced, whereby it is possible to prevent heat generation due to a short circuit of the crushed secondary battery more reliably. Accordingly, with the control program according to the present disclosure, it is possible to prevent the separator in the crushed secondary battery from melting and prevent the melted separator from sticking to the electrode material, and further prevent oxidation of the powdery crushed objects of the secondary battery, whereby it is possible to accurately sort the crushed objects of the secondary battery. That is, with the control program according to the present disclosure, it is possible to accurately sort the crushed objects of the secondary battery and efficiently recycle the crushed objects while reducing emission of CO2 by non-torrefaction.

According to the present disclosure, it is possible to provide a battery recycling system, a control method, and a control program capable of accurately sorting crushed objects of a secondary battery and efficiently recycling them while reducing emission of CO2 by non-torrefaction.

The above and other objects, features and advantages of the present disclosure will become more fully understood from the detailed description given hereinbelow and the accompanying drawings.

### BRIEF DESCRIPTION OF DRAWINGS

Fig. 1 is a block diagram showing a configuration example of a battery recycling system according to the present disclosure;
Fig. 2 is a flowchart showing a flow of processing of the battery recycling system according to the present disclosure;
Fig. 3 is a block diagram showing a specific example of an adjustment device provided in the battery recycling system according to the present disclosure;
Fig. 4 is a diagram showing a specific example of a primary crushing device provided in the battery recycling system according to the present disclosure;
Fig. 5 is a diagram showing a specific example of an electrolytic solution recovery device provided in the battery recycling system according to the present disclosure;
Fig. 6 is a diagram showing a specific example of a secondary crushing device provided in the battery recycling system according to the present disclosure;
Fig. 7 is a diagram showing a specific example of a screen provided in the secondary crushing device according to the present disclosure; and
Fig. 8 is a diagram showing a specific example of a sorting device provided in the battery recycling system according to the present disclosure.

### DESCRIPTION OF EMBODIMENTS

Hereinafter, with reference to the drawings, specific embodiments to which the present disclosure is applied will be described in detail. However, the present disclosure is not limited to the following embodiments. Further, for the sake of clarity of the description, the following description and the drawings are omitted as appropriate.

### <First Example Embodiment>

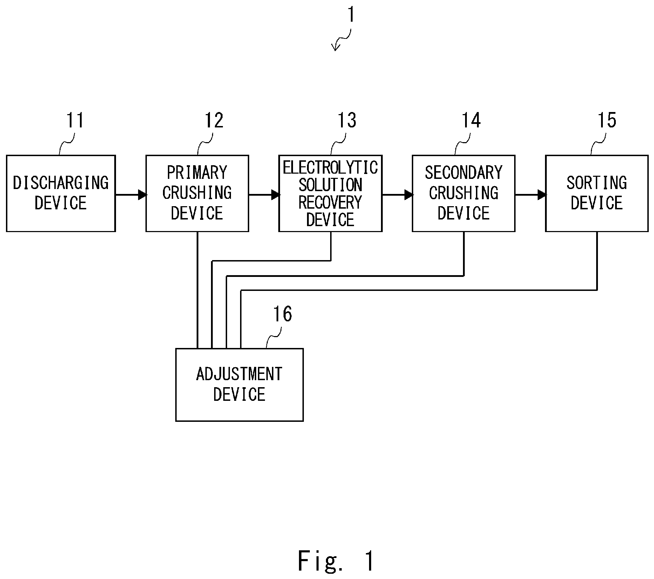

Fig. 1 is a block diagram showing a configuration example of a battery recycling system 1 according to a first example embodiment. The battery recycling system 1 is a system for crushing a secondary battery TG to be recycled and sorting crushed objects and so on for each material. The crushed objects and so on that have been sorted are recycled for a variety of applications. The battery recycling system 1 adjusts an atmosphere in each device to a nitrogen (N2) atmosphere and decreases an oxygen concentration, thereby preventing heat generation due to a short circuit of the crushed secondary battery more reliably. Accordingly, the battery recycling system 1 is able to prevent a separator in the crushed secondary battery from melting and prevent the melted separator from sticking to an electrode material, and further prevent oxidation of the powdery crushed objects of the secondary battery, whereby it is possible to accurately sort the crushed objects of the secondary battery. That is, the battery recycling system 1 can accurately sort the crushed objects of the secondary battery and efficiently recycle them while reducing emission of CO2 (carbon dioxide) due to non-torrefaction. Hereinafter, a specific explanation will be given.

As shown in Fig. 1, the battery recycling system 1 includes a discharging device 11, a primary crushing device 12, an electrolytic solution recovery device 13, a secondary crushing device 14, a sorting device 15, and an adjustment device 16. Note that processing in each of the discharging device 11, the primary crushing device 12, the electrolytic solution recovery device 13, the secondary crushing device 14, the sorting device 15, and the adjustment device 16 is performed in response to an instruction given by a control device (not shown). Note that a part of the processing in the discharging device 11, the primary crushing device 12, the electrolytic solution recovery device 13, the secondary crushing device 14, the sorting device 15, and the adjustment device 16 may be performed by a user operation. For example, processing for discharging the secondary battery by the discharging device 11 and introduction of the discharged secondary battery into the primary crushing device 12 may be performed by a user operation.

The discharging device 11 is a device that discharges the secondary battery TG to be recycled. The discharging device 11 connects, for example, the secondary battery TG to a predetermined resistance element, thereby discharging the secondary battery TG. The discharging device 11 may be a tray that is placed until when the secondary battery TG is completely discharged.

The secondary battery TG to be recycled is, for example, a lithium-ion battery or a nickel metal hydride battery. The secondary battery TG includes an electrode material and a case that accommodates the electrode material. The electrode material includes a negative electrode, a positive electrode, a separator provided therebetween, and an electrolytic solution.

In a lithium-ion battery, a case is formed of, for example, Al (aluminum). Cu (copper) is used for a current collector of the negative electrode, and graphite (C) is used for an active material of the negative electrode. Al is used for a current collector of the positive electrode, and a metallic compound containing Li (lithium) is used for an active material of the positive electrode. An organic solvent such as ethylene carbonate or propylene carbonate, and an electrolyte such as lithium salt are used for the electrolytic solution. A resin such as polyethylene or polypropylene is used for the separator. Further, Fe (iron) or the like is, for example, used for a bolt that fixes the electrode material accommodated in the case.

In a nickel metal hydride battery, the case is formed of a resin or the like. A hydrogen absorbing alloy is used for the negative electrode, and nickel hydroxide is used for the positive electrode. A potassium hydroxide solution is used for the electrolytic solution. A resin such as polyethylene or polypropylene is used for the separator. Further, Fe or the like is used for the external terminal.

The primary crushing device 12 crushes the case of the discharged secondary battery TG to cause the electrode material of the secondary battery TG to be exposed. Accordingly, the electrolytic solution contained in the electrode material can be recovered in the electrolytic solution recovery device 13 provided in the subsequent stage. Further, the primary crushing device 12 cuts the sheet-like separator, which is one of components of the electrode material, into small pieces. Accordingly, entanglement of the separator in the device provided in the subsequent stage, which may occur when the separator is not cut into small pieces, can be reduced, whereby degradation of the processing performance in the device provided in the subsequent stage can be suppressed.

The electrolytic solution recovery device 13 heats the crushed objects of the secondary battery TG (also referred to as the crushed objects TG) crushed by the primary crushing device 12 under a reduced-pressure environment, thereby distilling the electrolytic solution contained in the crushed objects TG and recovering the distilled electrolytic solution. Accordingly, the battery recycling system 1 can prevent heat generation due to a short circuit of the crushed secondary battery TG, whereby it is possible to prevent the separator in the crushed secondary battery TG from melting and prevent the melted separator from sticking to the electrode material. As a result, the battery recycling system 1 can prevent degradation in the accuracy of sorting the crushed objects generated by crushing the secondary battery TG.

The secondary crushing device 14 further crushes the crushed objects of the secondary battery TG (also referred to as the crushed objects TG) after the electrolytic solution is recovered. It is sufficient that the primary crushing device 12 provided in the former stage crush the secondary battery TG to such an extent that the electrode material of the secondary battery TG is exposed and the sheet-like separator is cut into small pieces. On the other hand, the secondary crushing device 14 crushes the crushed objects of the secondary battery TG after the electrolytic solution is recovered even more finely. Accordingly, the battery recycling system 1 can finely sort the crushed objects of the secondary battery TG for each material.

The sorting device 15 finely sorts the crushed objects of the secondary battery TG (also referred to as the crushed objects TG) crushed by the secondary crushing device 14 for each material. For example, the sorting device 15 finely sorts the crushed objects TG for each material using a plurality of sieves having different openings.

The adjustment device 16 adjusts the atmosphere in each of the primary crushing device 12, the electrolytic solution recovery device 13, the secondary crushing device 14, and the sorting device 15 to an N2 atmosphere, thereby setting the oxygen concentration in each device to be equal to or smaller than an acceptable value. Specifically, the adjustment device 16 may set the oxygen concentration in each of the primary crushing device 12 and the electrolytic solution recovery device 13 to be equal to or smaller than 2%, and set the oxygen concentration in each of the secondary crushing device 14 and the sorting device 15 to be equal to or smaller than 6%. Alternatively, the adjustment device 16 may uniformly set the oxygen concentration in each of the devices 12-15 to be equal to or smaller than 2%.

With the above structure, even in a case in which the electrolytic solution remains in the crushed secondary battery TG in each of the devices 12-15, the battery recycling system 1 can prevent heat generation due to a short circuit of the crushed secondary battery TG more reliably. Accordingly, the battery recycling system 1 is able to prevent the separator in the crushed secondary battery TG from melting and prevent the melted separator from sticking to the electrode material, and further prevent oxidation of the powdery crushed objects of the secondary battery TG, whereby it is possible to accurately sort the crushed objects of the secondary battery TG. That is, the battery recycling system 1 can accurately sort the crushed objects of the secondary battery TG and efficiently recycle the crushed objects while reducing emission of CO2 by non-torrefaction.

Preferably, the battery recycling system 1 does not start the operation of the devices 12-15 until when the oxygen concentration in each of the devices 12-15 becomes equal to or smaller than the acceptable value. When the oxygen concentration in any one of the devices 12-15 has exceeded the acceptable value after the start of the operation of the devices 12-15, the battery recycling system 1 may stop the operation of a device whose oxygen concentration has exceeded the acceptable value or all the devices 12-15. Further, the battery recycling system 1 may include an output device that outputs information indicating that the oxygen concentration in any one of the devices 12-15 has exceeded the acceptable value if the oxygen concentration in any one of the devices 12-15 has exceeded the acceptable value. The output device outputs, for example, a voice indicating that the oxygen concentration in any one of the devices 12-15 has exceeded the acceptable value from a speaker or displays this information on a monitor. The battery recycling system 1 may further include an analysis device that analyzes a degree of adhesion of the separator to the electrode material due to the melting of the separator from, for example, an image captured by a camera, and the adjustment device 16 may be configured to be able to adjust, based on the result of the analysis in the analysis device, the acceptable value of the oxygen concentration set in each of the devices 12-15. For example, when the degree of adhesion of the separator to the electrode material is high, the adjustment device 16 may set the acceptable value of the oxygen concentration in each of the devices 12-15 to be low.

### (Flow of Processing of Battery Recycling System 1)

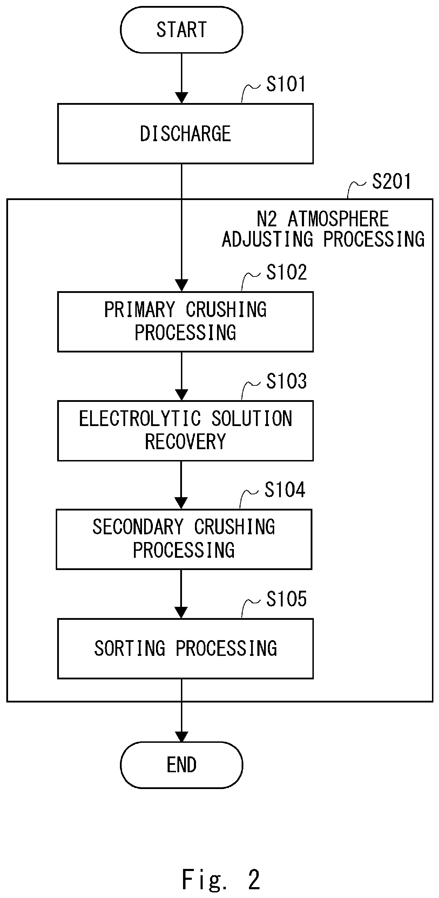

Next, with reference to Fig. 2, a flow of processing of the battery recycling system 1 will be described. Fig. 2 is a flowchart showing a flow of processing of the battery recycling system 1.

First, the battery recycling system 1 discharges the secondary battery TG to be recycled using the discharging device 11 (Step S101).

After that, the battery recycling system 1 performs primary crushing of the discharged secondary battery TG using the primary crushing device 12 (Step S102). Specifically, the battery recycling system 1 crushes the case of the discharged secondary battery TG to cause the electrode material of the secondary battery TG to be exposed and cuts the sheet-like separator, which is one of the components of the electrode material, into small pieces.

After that, the battery recycling system 1 heats the crushed objects of the secondary battery TG (crushed objects TG) crushed by the primary crushing device 12 under a reduced-pressure environment by using the electrolytic solution recovery device 13, thereby distilling the electrolytic solution contained in the crushed objects TG and recovering the distilled electrolytic solution (Step S103).

After that, the battery recycling system 1 crushes the crushed objects of the secondary battery TG (crushed objects TG) after the electrolytic solution is recovered even more finely using the secondary crushing device 14 (Step S104).

After that, the battery recycling system 1 sorts the crushed objects of the secondary battery TG (crushed objects TG) crushed by the secondary crushing device 14 for each material using the sorting device 15 (Step S105).

Here, the battery recycling system 1 adjusts the atmosphere in each of the primary crushing device 12 where primary crushing is performed, the electrolytic solution recovery device 13 where the electrolytic solution is recovered, the secondary crushing device 14 where secondary crushing is performed, and the sorting device 15 where sorting processing is performed to the N2 atmosphere, thereby setting the oxygen concentration in each of the devices 12-15 to be equal to or smaller than the acceptable value (Step S201).

With the above structure, the battery recycling system 1 is able to prevent heat generation due to a short circuit of the crushed secondary battery TG more reliably even in a case in which the electrolytic solution remains in the crushed secondary battery TG in each of the devices 12-15. Accordingly, the battery recycling system 1 is able to prevent the separator in the crushed secondary battery TG from melting and prevent the melted separator from sticking to the electrode material, and further prevent oxidation of the powdery crushed objects of the secondary battery TG, whereby it is possible to accurately sort the crushed objects of the secondary battery TG. That is, the battery recycling system 1 can accurately sort the crushed objects of the secondary battery TG and efficiently recycle the crushed objects while reducing emission of CO2 by non-torrefaction.

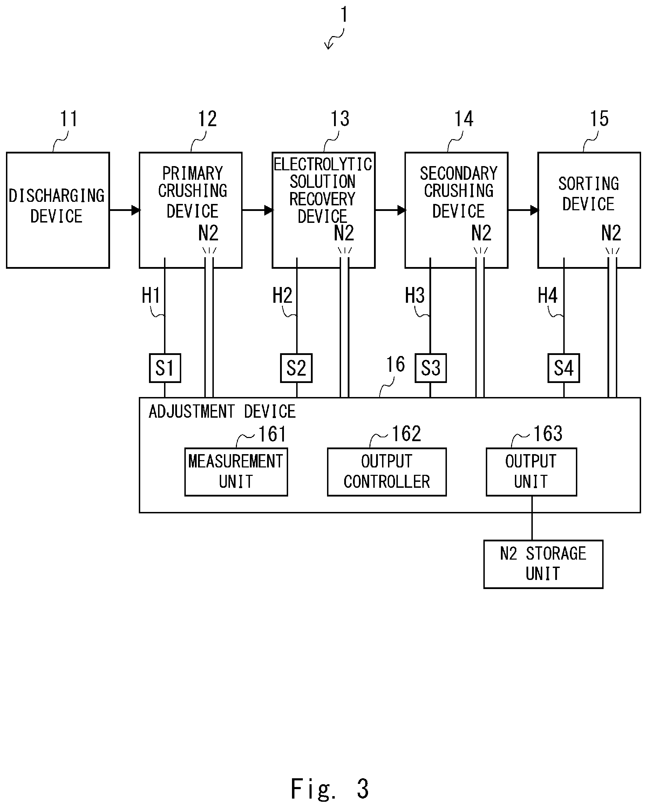

Hereinafter, with reference to Figs. 3-9, specific examples of each of the devices 12-15 will be described.

### (Specific Example of Adjustment Device 16 and its Peripheral Devices)

Fig. 3 is a block diagram showing a specific example of the adjustment device 16. Fig. 3 shows devices 11-15 other than the adjustment device 16 in the battery recycling system 1.

As shown in Fig. 3, the adjustment device 16 includes a measurement unit 161, an output controller 162, and an output unit 163. Further, hoses H1-H4 are attached to the primary crushing device 12, the electrolytic solution recovery device 13, the secondary crushing device 14, and the sorting device 15, respectively, so that the gas inside the respective devices passes through the hoses H1-H4. Further, oxygen concentration sensors S1-S4 are attached to respective gas outlets of the hoses H1-H4. Each of the oxygen concentration sensors S1-S4 is, for example, a zirconia type oxygen concentration sensor and generates a current or an electromotive force whose value corresponds to the oxygen concentration. Note that each of the oxygen concentration sensors S1-S4 is not limited to a zirconia type oxygen concentration sensor, and may be, for example, other type of oxygen concentration sensor such as a magnetic type, electrode type, or laser spectral type oxygen concentration sensor.

The measurement unit 161 computes the oxygen concentration in each of the devices 12-15 from the results of detection in the oxygen concentration sensors S1-S4. The output unit 163 supplies N2 (nitrogen) stored in an N2 storage unit to the inside of each of the devices 12-15. Along with the supply of N2 to the inside of each of the devices 12-15 by the output unit 163, the air inside each of the devices 12-15 is discharged outside the devices 12-15 via a scrubber. The output controller 162 controls the amount of N2 supplied to the inside of each of the devices 12-15 by the output unit 163 based on the oxygen concentration in each of the devices 12-15. For example, the output controller 162 controls the amount of N2 supplied to the inside of each of the devices 12-15 in such a way that the oxygen concentration in each of the primary crushing device 12 and the electrolytic solution recovery device 13 is maintained to be equal to or smaller than 2% and the oxygen concentration in each of the secondary crushing device 14 and the sorting device 15 is maintained to be equal to or smaller than 6%. Alternatively, the output controller 162 controls the amount of N2 supplied to the inside of each of the devices 12-15 in such a way that the oxygen concentration in the inside of each of the devices 12-15 is uniformly maintained to be equal to or smaller than 2%. Accordingly, even when the electrolytic solution remains in the crushed secondary battery TG in each device, the battery recycling system 1 can prevent heat generation due to a short circuit of the crushed secondary battery TG more reliably.

### (Specific Example of Primary Crushing Device 12)

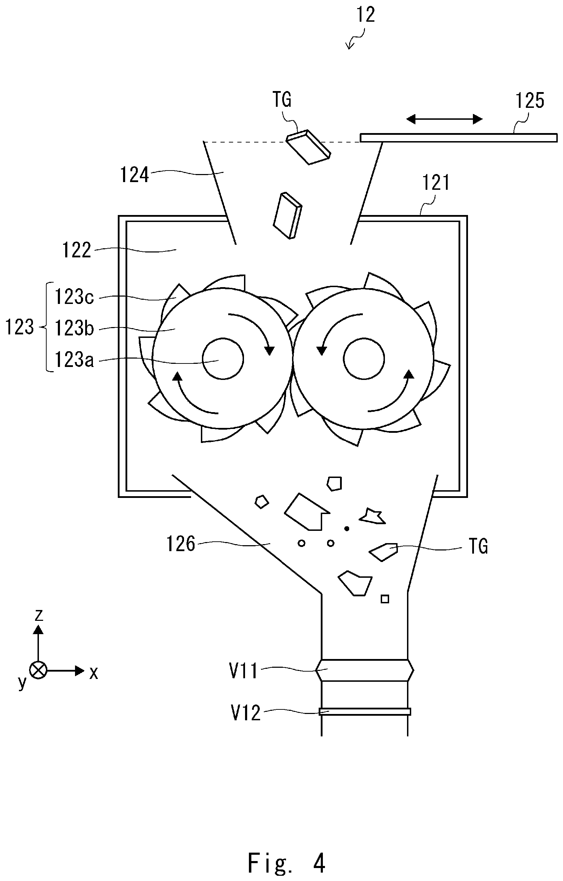

Fig. 4 is a diagram showing a specific example of the primary crushing device 12. As shown in Fig. 4, the primary crushing device 12 includes a main body 121, a crushing chamber 122, a crushing part 123, an inlet 124, an upper lid 125, and an outlet 126. Further, a crushing valve V11 that controls the passage of the crushed objects and a vacuum gate valve V12 that controls the passage of gas are provided on a route from the outlet 126 of the primary crushing device 12 to an inlet (not shown) of the electrolytic solution recovery device 13 provided in the subsequent stage.

The crushing chamber 122 is provided in the main body 121. The inlet 124 is provided above the main body 121 and communicates with the crushing chamber 122 in the main body 121. The outlet 126 is provided below the main body 121 and communicates with the crushing chamber 122 in the main body 121.

For example, first, in a state in which each of the crushing valve V11 and the vacuum gate valve V12 is closed, the upper lid 125 attached to the inlet 124 of the primary crushing device 12 is opened. After that, discharged secondary batteries TG are charged into the crushing chamber 122 via the inlet 124. Further, at this time, the oxygen concentration in the crushing chamber 122 is adjusted by the adjustment device 16 to be equal to or below an acceptable value (e.g., 2%). After that, the upper lid 125 is closed. Then, the crushing part 123 is driven by a motor (not shown), whereby the secondary batteries TG charged into the crushing chamber 122 start to be crushed by the crushing part 123.

The crushing part 123 is provided in the crushing chamber 122. The crushing part 123 includes a pair of rotating shafts 123a, a pair of rotors 123b, and a plurality of hooks 123c provided in the outer peripheral portion of each of the pair of rotors 123b. The pair of rotating shafts 123a are rotated in response to a driving force from a motor (not shown). Accordingly, the pair of rotors 123b that are attached to the pair of respective rotating shafts 123a and are arranged so as to be opposed to each other also rotate. In the example shown in Fig. 4, when seen from a y-axis positive direction, the rotor 123b on the left side rotates clockwise and the rotor 123b on the right side rotates counterclockwise. The secondary batteries TG charged into the crushing chamber 122 are guided between the pair of rotors 123b by the plurality of hooks 123c provided in the outer peripheral portion of each of the pair of rotors 123b, and are crushed by being pressed flat by the pair of rotors 123b. Note that the plurality of hooks 123c may be formed in a shape of a blade in such a way that a case and a separator of each of the secondary batteries TG are cut into small pieces.

The crushed objects of the secondary battery TG (crushed objects TG) crushed by the crushing part 123 fall into the outlet 126 provided below the crushing chamber 122. After the secondary batteries TG are crushed by the crushing part 123 and each of the crushing valve V11 and the vacuum gate valve V12 is opened, the crushed objects of the secondary batteries TG crushed by the crushing part 123 are discharged from the outlet 126.

### (Specific Example of Electrolyte Recovery Device 13)

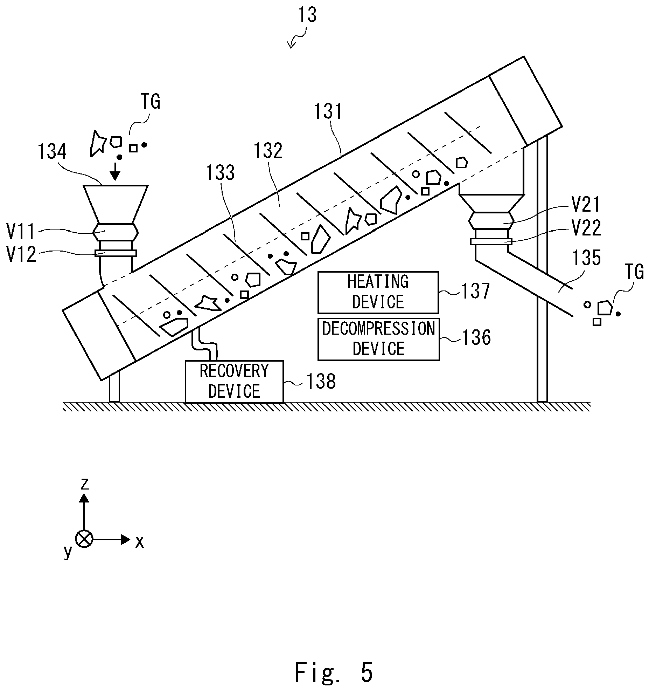

Fig. 5 is a diagram showing a specific example of the electrolytic solution recovery device 13. As shown in Fig. 5, the electrolytic solution recovery device 13 includes a main body 131, a conveyance chamber 132, a screw feeder 133, an inlet 134, an outlet 135, a decompression device 136, a heating device 137, and a recovery device 138. Further, as described above, the crushing valve V11 that controls the passage of the crushed objects and the vacuum gate valve V12 that controls the passage of gas are provided on a route from the outlet (not shown) of the primary crushing device 12 provided in the former stage to the inlet 134 of the electrolytic solution recovery device 13. Further, a crushing valve V21 that controls the passage of the crushed objects and a vacuum gate valve V22 that controls the passage of gas are provided on a route from the outlet 135 of the electrolytic solution recovery device 13 to an inlet (not shown) of the secondary crushing device 14 provided in the subsequent stage.

The conveyance chamber 132 is provided in a tube of the cylindrical main body 131. The inlet 134 is provided in a front end part of the cylindrical main body 131 and communicates with the conveyance chamber 132 in the main body 131. The outlet 135 is provided in a rear end part of the cylindrical main body 131 and communicates with the conveyance chamber 132 in the main body 131. The screw feeder 133 is provided in the conveyance chamber 132. The screw feeder 133 is driven by a motor (not shown), and conveys the crushed objects TG charged into the conveyance chamber 132 from the front end of the conveyance chamber 132 to the rear end thereof while stirring them by rotation. Accordingly, the crushed objects TG are located uniformly from the front end of the conveyance chamber 132 to the rear end thereof.

For example, first, each of the crushing valve V11 and the vacuum gate valve V12 provided on the side of the inlet 134 is opened in a state in which each of the crushing valve V21 and the vacuum gate valve V22 provided on the side of the outlet 135 is closed. After that, the crushed objects of the secondary battery TG (crushed objects TG) crushed by the primary crushing device 12 are charged into the conveyance chamber 132 via the inlet 134. The crushed objects TG charged into the conveyance chamber 132 are conveyed from the front end of the conveyance chamber 132 to the rear end thereof by the rotation of the screw feeder 133, and located uniformly from the front end of the conveyance chamber 132 to the rear end thereof. At this time, the oxygen concentration in the conveyance chamber 132 is adjusted by the adjustment device 16 to be equal to or below an acceptable value (e.g., 2%). After that, each of the crushing valve V11 and the vacuum gate valve V12 is closed. Then, the electrolytic solution contained in the crushed objects TG in the conveyance chamber 132 is recovered.

The decompression device 136 decompresses the conveyance chamber 132. The heating device 137 applies heat to the crushed objects TG in the conveyance chamber 132 under a reduced-pressure environment. Accordingly, the electrolytic solution contained in the crushed objects TG in the conveyance chamber 132 is vaporized. The recovery device 138 cools and liquefies the electrolytic solution vaporized in the conveyance chamber 132, thereby recovering the electrolytic solution. That is, the electrolytic solution recovery device 13 distills the electrolytic solution contained in the crushed objects TG in the conveyance chamber 132 and recovers the distilled electrolytic solution.

When, for example, the secondary battery TG is a lithium-ion battery, the electrolytic solution recovery device 13 recovers DMC, EMC, DEC, PC, and EC as the electrolytic solution. DMC stands for dimethyl carbonate. EMC stands for ethyl methyl carbonate. DEC stands for diethyl carbonate. PC stands for propylene carbonate. EC stands for ethylene carbonate. Further, when the secondary battery TG is a nickel metal hydride battery, the electrolytic solution recovery device 13 recovers water as the electrolytic solution.

Here, the electrolytic solution recovery device 13 may recover different kinds of electrolytic solutions by heating the crushed objects TG at different pressures and temperatures. When, for example, the secondary battery TG is a lithium-ion battery, the electrolytic solution recovery device 13 heats the crushed objects TG at a pressure lower than that when DMC, EMC, and DEC are recovered, thereby recovering PC and EC.

After the electrolytic solution is recovered and each of the crushing valve V21 and the vacuum gate valve V22 is opened, the crushed objects TG in the conveyance chamber 132 after the electrolytic solution is recovered are conveyed to the rear end of the conveyance chamber 132 by the rotation of the screw feeder 133 and then discharged from the outlet 135.

### (Specific Example of Secondary Crushing Device 14)

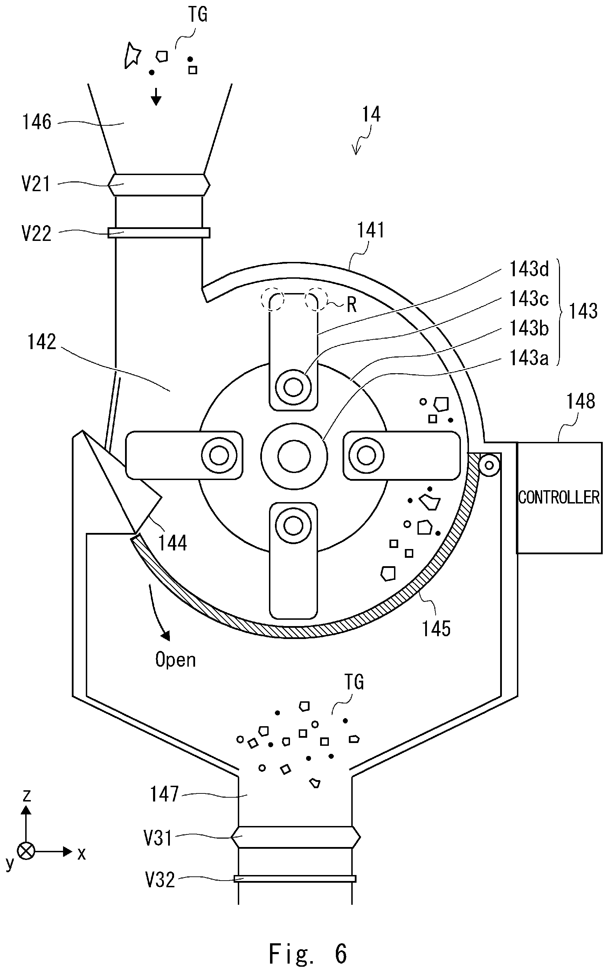

Fig. 6 is a diagram showing a specific example of the secondary crushing device 14. As shown in Fig. 6, the secondary crushing device 14 includes a main body 141, a crushing chamber 142, a crushing part 143, a fixed blade 144, a screen 145, an inlet 146, an outlet 147, and a controller 148. Further, as already described above, the crushing valve V21 that controls the passage of the crushed objects and the vacuum gate valve V22 that controls the passage of gas are further provided on a route from the outlet (not shown) of the electrolytic solution recovery device 13 provided in the former stage to the inlet 146 of the secondary crushing device 14. Further, a crushing valve V31 that controls the passage of the crushed objects and a vacuum gate valve V32 that controls the passage of gas are provided on a route from the outlet 147 of the secondary crushing device 14 to an inlet (not shown) of the sorting device 15.

The crushing chamber 142 is provided in the main body 141. The inlet 146 is provided above the main body 141 and communicates with the crushing chamber 142 in the main body 141. The outlet 147 is provided below the main body 141 and communicates with the crushing chamber 142 in the main body 141.

For example, first, each of the crushing valve V21 and the vacuum gate valve V22 provided on the side of the inlet 146 is opened in a state in which each of the crushing valve V31 and the vacuum gate valve V32 provided on the side of the outlet 147 is closed. After that, the crushed objects TG after the electrolytic solution is recovered are charged into the crushing chamber 142 via the inlet 146. Further, at this time, the oxygen concentration in the crushing chamber 142 is adjusted by the adjustment device 16 to be equal to or below an acceptable value (e.g., 6%). After that, each of the crushing valve V21 and the vacuum gate valve V22 is closed. Then the crushing part 143 is driven by a motor (not shown), whereby the crushed objects TG charged into the crushing chamber 142 start to be crushed by the crushing part 143.

The crushing part 143 and the fixed blade 144 are provided in the crushing chamber 142. The crushing part 143 is formed of a rotating shaft 143a, a rotor 143b, shafts 143c, and hammers 143d. The rotating shaft 143a rotates in response to a driving force from a motor (not shown). Accordingly, the rotor 143b attached to the rotating shaft 143a also rotates. A plurality of hammers (striking bodies) 143d are rotatably connected to an outer peripheral portion of the rotor 143b via the shaft 143c. Note that a plurality of rotors 143b may be provided in the axial direction (y-axis direction) of the rotating shaft 143a. In this case, the plurality of hammers 143d are rotatably connected to an outer peripheral portion of each rotor 143b via the shaft 143c. The hammers 143d rotate in the crushing chamber 142 by receiving a centrifugal force caused by the rotation of the rotating shaft 143a, and crush the crushed objects TG in the crushing chamber 142. The crushed objects TG are cut into small pieces between the fixed blade 144 and the rotating hammers 143d.

An edge portion of each hammer 143d is provided with an R (referred also to as an angle R). The angle R is provided at least at the tip of the hammer 143d, preferably at the entire portion of the outer peripheral portion of the hammer 143d that can hit the crushed objects TG when this hammer 143d is rotated. For example, the size of the angle R is determined based on a required size of the crushed objects TG after crushing, a required release rate of an active material of the crushed objects TG after crushing, or the like.

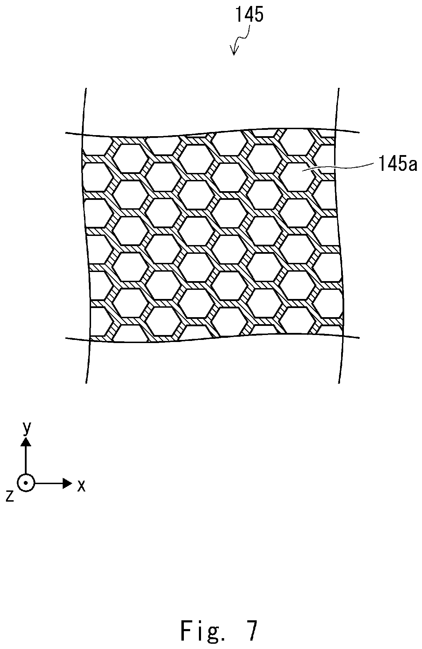

The screen 145 is provided at the bottom of the crushing chamber 142, and constitutes a part of the wall surface of the crushing chamber 142. The screen 145 allows crushed objects TG crushed by the crushing chamber 142 whose sizes are equal to or smaller than the size of an opening to pass. The screen 145 may be a mesh shape as shown in Fig. 7 or may be a punching metal. The opening of the screen 145 is set to be equal to or less than 5 mm, more preferably equal to or less than 1 mm. When the screen 145 has a mesh shape, the smallest width of the opening 145a is defined as the size of the opening. When the screen 145 is a punching metal including a plurality of circular opening parts, the opening is defined to be a diameter of the opening part.

The crushed objects TG crushed by the crushing part 143 or the like whose sizes are equal to or less than the opening pass through the screen 145 and fall into the outlet 147 provided below the crushing chamber 142. After each of the crushing valve V31 and the vacuum gate valve V32 is opened, the crushed objects TG are discharged from the outlet 147.

The controller 148 regularly opens the screen 145, thereby causing the crushed objects TG that cannot pass through the screen 145 and remain in the crushing chamber 142 to fall into the outlet 147. Accordingly, the crushed objects TG of the separator and so on that are light and are unlikely to pass through the screen 145 are discharged from the crushing chamber 142, which reduces the load on the processing of crushing the secondary crushing device 14.

Note that the controller 148 may separately discharge the crushed objects TG that have passed through the screen 145 and the crushed objects TG that cannot pass through the screen 145 and remain in the crushing chamber 142 from the outlet 147 at different timings. Further, the controller 148 may charge crushed objects obtained by removing light crushed objects of the separator and so on by a wind sorter or the like among the crushed objects TG that cannot pass through the screen 145 and remain in the crushing chamber 142 into the crushing chamber 142 again.

### (Specific Example of Sorting Device 15)

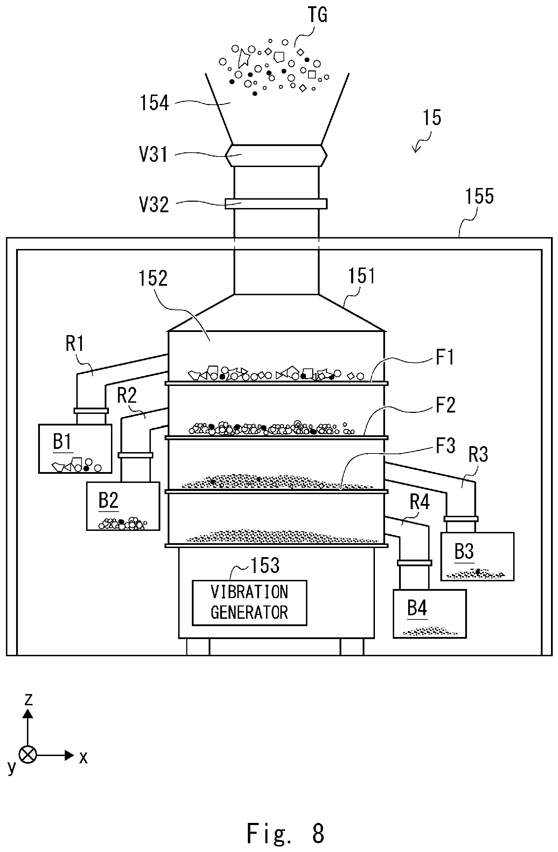

Fig. 8 is a diagram showing a specific example of the sorting device 15. As shown in Fig. 8, the sorting device 15 includes a main body 151, a sorting chamber 152, a vibration generator 153, an inlet 154, a box 155, recovery boxes B1-B4, sieves F1-F3, and routes R1-R4. Further, the crushing valve V31 that controls the passage of the crushed objects and the vacuum gate valve V32 that controls the passage of gas are provided on a route from the outlet (not shown) of the secondary crushing device 14 provided in the former stage to the inlet 154 of the sorting device 15, as described above.

The sorting chamber 152 is provided in the main body 151. The inlet 154 is provided above the main body 151, and communicates with the sorting chamber 152 in the main body 151.

For example, first, each of the crushing valve V31 and the vacuum gate valve V32 provided on the side of the inlet 154 is opened. After that, the crushed objects of the secondary battery TG (crushed objects TG) crushed by the secondary crushing device 14 are charged into the sorting chamber 152 via the inlet 154. Further, at this time, the oxygen concentration in the sorting chamber 152 is adjusted by the adjustment device 16 to be equal to or below an acceptable value (e.g., 6%). After that, each of the crushing valve V31 and the vacuum gate valve V32 is closed. Then, the crushed objects TG in the sorting chamber 152 are sorted.

The sieves F1-F3 are provided in the sorting chamber 152. The sieve F1 having the largest opening is provided at the top stage, the sieve F2 having the second largest opening is provided at the middle stage, and the sieve F3 having the smallest opening is provided at the bottom stage. The number of sieves is not limited to three, and a desired number of sieves may be provided. The vibration generator 153 vibrates the sieves F1-F3.

The crushed objects TG charged into the sorting chamber 152 are first sorted into crushed objects that have passed through the sieve F1 and crushed objects that have not passed through the sieve F1. The crushed objects TG that have not passed through the sieve F1 are discharged into the recovery box B1 via the route R1. The crushed objects TG that have passed through the sieve F1 are sorted into crushed objects that have passed through the sieve F2 and crushed objects that have not passed through the sieve F2. The crushed objects TG that have not passed through the sieve F2 are discharged into the recovery box B2 via the route R2. The crushed objects TG that have passed through the sieve F2 are sorted into crushed objects that have passed through the sieve F3 and crushed objects that have not passed through the sieve F3. The crushed objects TG that have not passed through the sieve F3 are discharged into the recovery box B3 via the route R3. Further, the crushed objects TG that have passed through the sieve F3 are discharged into the recovery box B4 via the route R4.

The recovery box B1 recovers, for example, crushed objects of a separator, an aluminum case, or the like. The recovery box B2 recovers, for example, crushed objects of an Al foil, a Cu foil, or the like. The recovery box B3 recovers, for example, a black mass containing a relatively large amount of impurities. The recovery box B4 recovers a black mass with a small amount of impurities. The black mass indicates powdery crushed objects such as an active material contained in the secondary battery. In a case of a lithium-ion battery, the black mass contains Ni, Co, Mn, Li, C, and so on. In a case of a nickel metal hydride battery, the black mass contains Ni, Co, La, Zn, and so on.

Note that the sorting device 15 is accommodated in the box 155. By supplying N2 into the box 155 by the adjustment device 16, the oxygen concentration in not only the sorting chamber 152 but also an area near the main body 151 is reduced, whereby oxidation of the black mass or the like sorted by the sorting device 15 is suppressed.

While the case in which the sorting device 15 sorts the crushed objects TG using a plurality of sieves having different openings has been described as an example, this is merely one example. For example, the sorting device 15 may sort the crushed objects TG using some or all of a wind sorter, a magnetic separator, an optical sorter, a dry specific gravity sorter, and the aforementioned sieve sorter.

For example, the wind sorter blows wind onto the crushed objects TG, thereby sorting the crushed objects TG into crushed objects made of a light material blown up by wind and crushed objects made of other materials. The magnetic separator sorts the crushed objects TG into crushed objects such as iron attracted to magnets and other crushed objects. The optical sorter determines the materials of the crushed objects TG based on the colors thereof specified from an image captured by a camera and blows, for example, air whose wind power corresponds to the result of the determination onto the crushed objects TG, thereby sorting the crushed objects TG. The dry specific gravity sorter vibrates a tray charged with the crushed objects TG, thereby sorting the crushed objects TG charged into the tray into crushed objects made of a material with light specific gravity and crushed objects made of a material with a heavy specific gravity.

As described above, the battery recycling system 1 according to the present disclosure adjusts the atmosphere in each of the devices 12-15 to the N2 atmosphere and decreases the oxygen concentration, thereby preventing heat generation due to a short circuit of the crushed secondary battery more reliably. Accordingly, the battery recycling system 1 according to the present disclosure is able to prevent the separator in the crushed secondary battery from melting and prevent the melted separator from sticking to the electrode material, and further prevent oxidation of the powdery crushed objects of the secondary battery, whereby it is possible to accurately sort the crushed objects of the secondary battery. That is, the battery recycling system 1 according to the present disclosure is able to accurately sort the crushed objects of the secondary battery and efficiently recycle the crushed objects while reducing emission of CO2 by non-torrefaction.

While the case in which the battery recycling system 1 crushes the secondary battery TG and sorts the crushed objects and so on of the secondary battery TG for recycling is described as an example in the present disclosure, this is merely an example. For example, an all solid-state battery or the like may be crushed and its crushed objects and so on may be sorted for recycling.

The battery recycling system 1 may further include an analysis device that analyzes the content of the black mass and a management device that adjusts operation conditions of each of the devices 12-15 (e.g., the crushing time, the crushing power and the like of each of the primary crushing device and the secondary crushing device) in such a way that the analysis result in the analysis device becomes a desired analysis result.

Further, the present disclosure can implement some or all of the processing of the battery recycling system 1 by causing a Central Processing Unit (CPU) to execute a computer program.

The aforementioned program includes instructions (or software codes) that, when loaded into a computer, cause the computer to perform one or more of the functions described in the embodiments. The program may be stored in a nontransitory computer readable medium or a tangible storage medium. By way of example, and not a limitation, computer readable media or tangible storage media can include a random-access memory (RAM), a read-only memory (ROM), a flash memory, a solid-state drive (SSD) or other types of memory technologies, a CD-ROM, a digital versatile disc (DVD), a Blu-ray (registered trademark) disc or other types of optical disc storage, and magnetic cassettes, magnetic tape, magnetic disk storage or other types of magnetic storage devices. The program may be transmitted on a transitory computer readable medium or a communication medium. By way of example, and not a limitation, transitory computer readable media or communication media can include electrical, optical, acoustical, or other forms of propagated signals.

While the present disclosure has been described above with reference to the embodiments, the present disclosure is not limited to the above-described embodiments. Various changes that can be understood by those skilled in the art within the scope of the present disclosure can be made to the configurations and the details of the present application. Each of the embodiments can be combined with another embodiment as necessary.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A battery recycling system (1) comprising:
a discharging device (11) configured to discharge a secondary battery (TG) to be recycled;
a primary crushing device (12) configured to crush a case of the secondary battery that has been discharged to cause an electrode material of the secondary battery to be exposed;
an electrolytic solution recovery device (13) configured to heat the secondary battery whose electrode material has been exposed under a reduced-pressure environment to recover an electrolytic solution contained in the electrode material;
a secondary crushing device (14) configured to further crush the secondary battery whose electrolytic solution has been recovered;
a sorting device (15) configured to sort crushed objects of the secondary battery crushed by the secondary crushing device (14); and
an adjustment device (16) configured to adjust an atmosphere in each of the primary crushing device (12), the electrolytic solution recovery device (13), the secondary crushing device (14), and the sorting device (15) to an N2 atmosphere.

2. The battery recycling system according to claim 1, wherein the adjustment device (16) adjusts an amount of N2 supplied in such a way that the oxygen concentration in each of the primary crushing device (12), the electrolytic solution recovery device (13), the secondary crushing device (14), and the sorting device (15) becomes equal to or smaller than 2%.

3. The battery recycling system according to claim 1, wherein the adjustment device (16) adjusts an amount of N2 supplied in such a way that the oxygen concentration in each of the primary crushing device (12) and the electrolytic solution recovery device (13) becomes equal to or small than 2% and the oxygen concentration in each of the secondary crushing device (14) and the sorting device (15) becomes equal to or smaller than 6%.

4. The battery recycling system according to claim 1, wherein each of the primary crushing device (12), the electrolytic solution recovery device (13), the secondary crushing device (14), and the sorting device (15) starts an operation when the oxygen concentration therein has been adjusted to be the one required by the adjustment device (16).

5. The battery recycling system according to claim 1, wherein the secondary battery is a lithium-ion battery or a nickel metal hydrate battery.

6. A control method of a battery recycling system (1), wherein
the battery recycling system:
discharges, using a discharging device (11), a secondary battery (TG) to be recycled;
crushes, using a primary crushing device (12), a case of the secondary battery that has been discharged to cause an electrode material of the secondary battery to be exposed;
heats, using an electrolytic solution recovery device (13), the secondary battery whose electrode material has been exposed under a reduced-pressure environment to recover an electrolytic solution contained in the electrode material;
further crushes, using a secondary crushing device (14), the secondary battery whose electrolytic solution has been recovered; and
sorts, using a sorting device (15), crushed objects of the secondary battery crushed by the secondary crushing device (14), and
an atmosphere in each of the primary crushing device (12), the electrolytic solution recovery device (13), the secondary crushing device (14), and the sorting device (15) is adjusted to an N2 atmosphere.

7. A control program for causing a computer to execute:
processing for discharging, using a discharging device (11), a secondary battery (TG) to be recycled;
processing for crushing, using a primary crushing device (12), a case of the secondary battery that has been discharged to cause an electrode material of the secondary battery to be exposed;
processing for heating, using an electrolytic solution recovery device (13), the secondary battery whose electrode material has been exposed under a reduced-pressure environment to recover an electrolytic solution contained in the electrode material;
processing for further crushing, using a secondary crushing device (14), the secondary battery whose electrolytic solution has been recovered; and
processing for sorting, using a sorting device (15), crushed objects of the secondary battery crushed by the secondary crushing device (14), and
the computer is caused to further execute processing for adjusting an atmosphere in each of the primary crushing device (12), the electrolytic solution recovery device (13), the secondary crushing device (14), and the sorting device (15) to an N2 atmosphere.
